# EUROPEAN PATENT SPECIFICATION

(11) **EP 1 404 323 B1**
(45) Date of publication and mention of the grant of the patent: **28.10.2009**
(21) Application number: 02741899.5
(22) Date of filing: 05.06.2002
(51) Int. Cl.: A61K 31/44, A61K 31/485, A61P 37/04

(54) **USE OF METHYLNALTREXONE TO TREAT IMMUNE SUPPRESSION**
VERWENDUNG VON METHYLNALTREXON ZUR BEHANDLUNG VON IMMUNSUPPRESSION
UTILISATION DE LA METHYLNALTREXONE POUR LE TRAITEMENT DE L'IMMUNODEPRESSION

(30) Priority: 05.06.2001 US 295571 P; 22.04.2002 US 374454 P
(43) Date of publication of application: 07.04.2004
(73) Proprietor: The University of Chicago, Chicago, IL 60637 (US)
(72) Inventor: MOSS, Jonathan, Chicago, IL 60637 (US); YUAN, Chun-Su, Chicago, IL 60637 (US)
(74) Representative: Hutter, Anton
(86) International application number: PCT/US2002/018087
(87) International publication number: WO 2002/098422

(56) References cited:
- EP-A- 0 984 004
- WO-A-01/13909
- WO-A-01/37785
- WO-A-96/23793
- WO-A-99/22737
- US-A- 4 176 186
- US-A- 5 102 887
- US-A- 5 270 328
- US-A- 5 958 452
- RADULOVIC J ET AL: "Opioid receptor-mediated suppression of humoral immune response in vivo and in vitro: involvement of kappa opioid receptors." JOURNAL OF NEUROIMMUNOLOGY. MAR 1995, vol. 57, no. 1-2, March 1995 (1995-03), pages 55-62, XP002395947 ISSN: 0165-5728
- JANKOVIC B D ET AL: "Quaternary naltrexone: its immunomodulatory activity and interaction with brain delta and kappa opioid receptors." IMMUNOPHARMACOLOGY. 1994 SEP-OCT, vol. 28, no. 2, September 1994 (1994-09), pages 105-112, XP002395948 ISSN: 0162-3109
- NELSON C J ET AL: "Involvement of central mu- but not delta- or kappa-opioid receptors in immunomodulation." BRAIN, BEHAVIOR, AND IMMUNITY. SEP 2000, vol. 14, no. 3, September 2000 (2000-09), pages 170-184, XP002395949 ISSN: 0889-1591
- MANCEV Z ET AL: "THE IMMUNOMODULATING EFFECTS OF SPECIFIC OPIOID RECEPTOR ANTAGONISTS AFTER THEIR INTRACEREBROVENTRICULAR APPLICATION" INTERNATIONAL JOURNAL OF THYMOLOGY, THYMUS MED. FACHBUCHVERLAG, BAD HARYBURG, DE, vol. 7, no. 12-13, 1999, pages 589-595, XP008065206 ISSN: 0943-1675
- LYSLE D T ET AL: "EVIDENCE FOR THE INVOLVEMENT OF THE CAUDAL REGION OF THE PERIAQUEDUCTAL GRAY IN A SUBSET OF MORPHINE-INDUCED ALTERATIONS OF IMMUNE STATUS" JOURNAL OF PHARMACOLOGY AND EXPERIMENTAL THERAPEUTICS, AMERICAN SOCIETY FOR PHARMACOLOGY AND, US, vol. 277, no. 3, 1996, pages 1533-1540, XP008065207 ISSN: 0022-3565
- MELLON R D ET AL: "Evidence for central opioid receptors in the immunomodulatory effects of morphine: Review of potential mechanism(s) of action" JOURNAL OF NEUROIMMUNOLOGY 15 MAR 1998 NETHERLANDS, vol. 83, no. 1-2, 15 March 1998 (1998-03-15), pages 19-28, XP002395950 ISSN: 0165-5728
- FECHO K ET AL: "ASSESSMENT OF THE INVOLVEMENT OF CENTRAL NERVOUS SYSTEM AND PERIPHERAL OPIOID RECEPTORS IN THE IMMUNOMODULATORY EFFECTS OF ACUTE MORPHINE TREATMENT IN RATS" JOURNAL OF PHARMACOLOGY AND EXPERIMENTAL THERAPEUTICS, AMERICAN SOCIETY FOR PHARMACOLOGY AND, US, vol. 276, no. 2, February 1996 (1996-02), pages 626-636, XP008065551 ISSN: 0022-3565
- YUAN C-S ET AL: "MYTHYLNALTREXONE FOR REVERSAL OF CONSTIPATION DUE TO CHRONIC METHADONE USE" JAMA THE JOURNAL OF THE AMERICAN MEDICAL ASSOCIATION, CHICAGO,IL, US, vol. 283, no. 3, 19 January 2000 (2000-01-19), pages 367-372, XP008065666 ISSN: 0098-7484
- NOVICK D M ET AL: "NATURAL KILLER CELL ACTIVITY AND LYMPHOCYTE SUBSETS IN PARENTERAL HEROIN ABUSERS AND LONG-TERM METHADONE MAINTENANCE PATIENTS" JOURNAL OF PHARMACOLOGY AND EXPERIMENTAL THERAPEUTICS, AMERICAN SOCIETY FOR PHARMACOLOGY AND, US, vol. 250, no. 2, August 1989 (1989-08), pages 606-610, XP008065247 ISSN: 0022-3565
- SZABO I ET AL: "INTERACTIONS OF OPIOID RECEPTORS, CHEMOKINES, AND CHEMOKINE RECEPTORS" ADVANCES IN EXPERIMENTAL MEDICINE AND BIOLOGY, SPRING ST., NY, US, vol. 493, 2001, pages 69-74, XP008065610 ISSN: 0065-2598
- MCCARTHY L ET AL: "Opioids, opioid receptors, and the immune response." DRUG AND ALCOHOL DEPENDENCE. 1 APR 2001, vol. 62, no. 2, 1 April 2001 (2001-04-01), pages 111-123, XP002395951 ISSN: 0376-8716
- FOSS J F ET AL: "Prevention of apomorphine- or cisplatin-induced emesis in the dog by a combination of methylnaltrexone and morphine." CANCER CHEMOTHERAPY AND PHARMACOLOGY. 1998, vol. 42, no. 4, 1998, pages 287-291, XP002395952 ISSN: 0344-5704
- MIYAGI T ET AL: "Morphine induces gene expression of CCR5 in human CEMx174 lymphocytes." THE JOURNAL OF BIOLOGICAL CHEMISTRY. 6 OCT 2000, vol. 275, no. 40, 6 October 2000 (2000-10-06), pages 31305-31310, XP002395953 ISSN: 0021-9258
- PETERSON P K ET AL: "MORPHINE PROMOTES THE GROWTH OF HIV-1 IN HUMAN PERIPHERAL BLOOD MONONUCLEAR CELL COCULTURES" AIDS, LONDON, GB, vol. 4, no. 9, September 1990 (1990-09), pages 869-873, XP008065616 ISSN: 0269-9370
- STEELE A D ET AL: "HIV-1 INFECTION AND OPIOID ADMINISTRATION MODULATE THE EXPRESSION OF CHEMOKINE RECEPTORS" DRUG AND ALCOHOL DEPENDENCE, ELSEVIER SCIENTIFIC PUBLISHERS, IR, vol. 60, no. SUPPL 1, 1 December 2000 (2000-12-01), page S212, XP008065558 ISSN: 0376-8716
- NAIR M P N ET AL: "MORPHINE MODULATES THE EXPRESSION OF CHEMOKINES AND THEIR RECEPTORS BY PERIPHERAL BLOOD MONONUCLEAR CELLS (PBMC) FROM NORMAL DONORS" JOURNAL OF ALLERGY AND CLINICAL IMMUNOLOGY, MOSBY - YEARLY BOOK, INC, US, vol. 101, no. 1, PART 2, January 1998 (1998-01), page S57, XP008065575 ISSN: 0091-6749
- HO ET AL.: JOURNAL OF PHARMACOLOGY AND EXPERIMENTAL THERAPEUTICS, vol. 307, 2003, pages 1158-1162,

## Description

### FIELD OF THE INVENTION

The invention relates to the field of treating immune suppression. In particular, the invention relates to the discovery that opioid-induced immune suppression is treatable by administration of peripheral opioid antagonists.

### BACKGROUND OF THE INVENTION

Opioid medications are widely used clinically for relieving pain, and as antidiartheals and antitussives. Opioid agonists consist of a group of natural, semisynthetic, or synthetic compounds acting on a series of receptors, such as mu-, kappa-, and delta-receptors. Concomitant with the ability to relieve pain, these drugs can have adverse effects. Side effects of opioid treatment include nausea, vomiting, respiratory suppression, fatigue, sweating, difficult micturation, constipation, psychomimetic disturbance, and dependence.

Another adverse side effect of opioid administration is immunosuppression. Although well described as a laboratory phenomenon in numerous case reports and clinical studies (Eisenstein, et al. J. Neuroimmunol (1998) 15, 83:36-44), its overall importance as an adverse effect of opioid use has not been fully understood by those of skill in the art. The exact mechanisms of action of opioid effects on the immune system are controversial, but several studies suggest that the immunomodulatory effect of opioids may be mediated via mechanisms that are different from those responsible for the antinociceptive effects.

Opioids induce immune suppression, as evidenced by animal and human studies. Both animals treated with opioids and heroin addicts have increased infection rates (Tubaro et al., J. Infect. Dis. (1983) 148, 656-66.; Risdahl et al., J. Infect. Dis. (1993) 167, 1281-7; Risdahl et al., J. Neuroimmunol (1998) 83, 4-18; Hussey, et al., Am. J. Med. (1950) 9, 186-93; Haverkos et al., J. Infect. Dis., (1990) 161, 894-912; Quinn, Emerg. Med. Clin. North Am. (1995), 13, 1-25). Narcotic abusers have greater incidence of infection than non-abusers. For example, narcotic addicts have a markedly increased prevalence of viral hepatitis, bacterial pneumonias, endocarditis, tuberculosis, soft tissue and CNS infections (Hussey *et al.,* (1950) *supra*; Louria et al., Ann. Int. Med. (1967) 67, 1-22; Reichman et al., Arch. Intern. Med. (1979) 139, 337-39; Haverkos (1990), *supra*).

Chronically exposed individuals show a series of changes in their ability to respond to immunological challenges. Narcotic addicts, as well as patients and animals receiving opioids, exhibit abnormalities in many immunological parameters including decreased natural killer (NK) cell cytolytic activity, blood lymphocyte proliferation responses to mitogen, and alterations in more complex immune responses including antibody-dependent cell-mediated cytotoxicity and antibody production (Layon et al., Arch Intern. Med (1984) 144, 1376-80; Nair et al., Clin. Immunol. Immunopathol. (1986) 38, 68-78; Molitor et al., J. Pharmacol Exp. Ther. (1991) 260, 581-6; Brown et al., Arch. Intern. Med. (1974) 134, 1001-6; Morgan, J. Neuoroimmunol (1996) 65(1), 21-30; Palm et al., Anesth. Analg. (1998) 86(1), 166-72).

In addition to the effects of opioids on chronic opioid users, opioids affect immunomodulation functions in healthy normal individuals exposed to opioids (Crone et al., Anesth Analg. (1988) 67, 318-23; Biagini et al., Arch Environ Health (1995) 50(1), 7-12). Opioids have been demonstrated to inhibit lymphocyte proliferation, decrease splenic lymphocyte number, and alter phenotypic expression of cell surface marker (Hamna et al., Anesthesiology (1996) 85(2), 355-65).

Opioids also have suppressive effects on hematopoietic cell development, resulting in atrophy of both the thymus and the spleen (Bryant et al., Life Sci. (1987) 41, 1731-8; Hilburger et al., J. Neuroimmunol. (1997) 80, 106-14; Frier et al., J. Pharmacol. Exp. Ther. (1993) 265; 81-8), and reduced numbers of macrophages and B-cells in the murine spleen (Singhal et al., J. Immunol. (1998) 160, 1886).

The immunosuppressive characteristics of opioids are increasingly important with the increase in patients infected with HIV virus and patients with Acquired Immune Deficiency Syndrome (AIDS). A large number of HIV-1 infected individuals are drug abusers and/or addicts, and there is a correlation between drug abuse and HIV infection (Swan, AIDS Res. (1997) 12, 2; Donahoe, Adv. Neuroimmunol (1993) 3, 3146); CDC, 1996, Vol. 7, No.2).

*In vitro,* it has been demonstrated that morphine promotes the growth of HIV-1 in human peripheral blood mononuclear cell cultures (Peterson et al., AIDS (1990) 4, 869-73; Chao et al., Biochem. Pharmacol. (1995) 50, 715-22). Although the mechanism of increased HIV load in opioid addicts is unclear, a recent study suggests a direct effect of opioids on CCR5 turnover. Li *et al.* demonstrated that methadone significantly enhanced HIV infection of macrophages with the up-regulation of expression of CCR5, a primary coreceptor for macrophage-tropic HIV entry into macrophages (Li et al., J. Infect. Dis. (2002) 185(1), 118-22). The relationship between opioid binding and CCR5 regulation remains to be determined.

*In vivo* inhibition of lymphocyte proliferation by morphine has been shown, and this inhibition was completely antagonized by naltrexone pretreatment, suggesting involvement of opioid receptors (Bayer et al. Immunopharmacology (1992) 23(2), 117-24). Yeager *et al.* observed that intravenous morphine inhibited NK cell cytotoxicity in volunteers (Yeager et al. Anesthesiology (1995) 83, 500-8).

The use of opioid antagonists as drugs to treat acquired immunodeficiency states (such as an HIV infection) was recognized by Shelly (Australian Patent No. 610,561). Shelley used central opioid antagonists such as naltrexone, and mentioned but disclosed no data on peripheral opioid antagonists, such as methylnaltrexone, to treat viral infections and acquired immunodeficiency states. Shelley did not address treating immunosuppression caused by the administration of opioids and teaches away from administering opioid antagonists to patients receiving opioids.

Although the mechanism of opioid-induced immunosuppression appears to be opioid receptor mediated, there are conflicting reports with regard to more specific mechanism. It has not been definitively shown which opioid receptor or receptors are involved in opioid-induced immunosuppression, as there is evidence implicating both mu- and kappa-opioid receptors. Additionally, it remains controversial as to whether the opioid receptor mediated immunosuppression operates via a central or peripheral mechanism.

An early study demonstrated that morphine injected into the lateral ventricle suppressed NK cell activity, and this immunosuppressive effect was blocked by naltrexone, a non-selective opioid antagonist (Shavit et al., Proc. Natl. Acad. Sci. (1986) 83, 7114-7).

Hernandez *et al.* further examined whether the immunosuppressive effect of morphine is mediated by opioid receptors located at either peripheral or central sites (Hernandez et al., J. Pharmacol. Exp. Ther. (1993) 267, 336-41). First, the effects of systemic morphine administration on analgesia, mitogen-stimulated lymphocyte proliferation and corticosterone secretion were compared to those observed after systemic administration ofN-methyl-morphine. N-methyl-morphine is a morphine analogue that does not cross the blood-brain barrier. In contrast to morphine, N-methyl-morphine did not effect lymphocyte proliferation, plasma corticosterone concentrations or analgesic responses, indicative of a centrally-mediated mechanism. Second, the effects of morphine and N-methyl-morphine after central administration were compared. With the microinjection of either morphine or N-methyl-morphine into the third ventricle, blood lymphocyte responses were inhibited by 70%; plasma corticosterone concentration were significantly elevated; and maximal analgesic responses were present. These data are also indicative of a centrally-mediated mechanism.

A recent finding greatly assisted the understanding of the mechanism of opioid-induced immunosuppression: with opioids, apoptosis (cell death) of immune cells is accelerated by directly inducing Fas (a death receptor) expression (Yin et al., Nature (1999) 397 (6716) 218). Subsequently, Yin *et al.* observed that stress modulates the immune system through CD95 (Fax/APO-1)-medicated apoptosis dependent on endogenous opioids. These investigators showed that chronically stressed mice exhibit a significant reduction in splenocytes (a process mediated by apoptosis) and an increase in CD95 expression. These stress-induced changes in lymphocyte number and CD95 expression were blocked by naloxone or naltrexone, centrally acting opioid antagonists (Yin *et al.,* (2000), *supra*). In addition, the reduction of splenocytes observed seems to be independent of the hypothalamic-pituitary-adrenal axis, since both adrenaletomized and sham-operated mice exhibited similar response to the chronic stress. These data point to a central mechanism of opioid-induced immunosuppression.

Several studies have attempted to identify the specific brain regions involved in opioid-induced immunoregulation (Gomez-Flores et al., Immunopharmacology (2000) 48(2), 145-56; Weber et al., Science (1989) 245, 188-90). In the brain, periaqueductal gray matter (PAG) serves a variety of diverse autonomic functions and appears to be a site for opioid action in the induction of immunosuppression. The PAG has been identified as a site of morphine-mediated naltrexone-sensitive suppression of rat splenic NK cell activity. Opioid receptors and endogenous opioid peptides are present in the PAG, and endogenous opioids are released in the PAG as a result of stress (Seeger et al., Brain Res. (1984) 305(2), 303-11). Additionally, microinjections of morphine into the PAG specifically result in a rapid suppression ofNK cell activity, and prior systemic administration of naltrexone can block NK cell suppression. These findings demonstrate that opioid- induced suppression ofNK cell function is mediated through opioid receptors in the PAG.

Immune system cells express mu, delta, and kappa receptors which are functionally coupled with signal transduction mechanisms. Several studies have found that central mu-receptors are involved in immunomodulation, but neither delta nor kappa opioid receptors are involved (Carr et al., Proc. Soc. Exp. Biol. Med (1996) 213, 248-57; Band et al., Prog. Neuro EndocrinImmunol. (1992) 5, 95-101; Nelson et al., Brain Behavor Immunity (2000) 14, 170-84; Mellon et al., Brain Res. (1998) 789(1), 56-67). Intracerebroventricular administration of the mu-selective opioid agonist DAMGO to rats increased splenocyte production of nitric oxide, a chemical linked to central immunomodulation; this effect was blocked by prior injection of a peripheral opioid antagonist (Schneider et al., J. Neuroimmunol. (1998) 89, 150-9). In contrast, intracerebroventricular administration of either a kappa-selective agonist (U69,593) or a delta-selective agonist (DPDPE) had no significant effect on the production of nitric oxide. Nowak *et al.* also demonstrated that injection of SNC 80, a nonpeptidic delta-opioid receptor-selective agonist, in rats did not affect splenic NK cell activity (Nowak et al. J. Pharmacol. Exp. Ther. (1988) 286(2), 931-7). These results point to a central mu-opioid receptor mediated mechanism of opioid-induced immunosuppression.

The findings that mu-opioid receptors within the central nervous system are responsible for the immune suppression induced by opioid was further supported by another animal study, in which comparable results were obtained after administration of mu, kappa, and delta agonists to the ventricle (Nelson *et al.*, *supra*). Involvement of central mu-receptors in immunosuppression has also been observed in a recent investigation in rats (Sacerdote et al., Int. Immunopharmacol. (2001) 1, 713-9). In the study, rats received remifentanil, a pure mu-receptor agonist with a half-life of only several minutes, which showed similar immunosuppressive effects to other mu-receptor agonists.

The centrally-mediated immunosuppressive effects of opioid administration have been mapped to mu-opioid receptors in the mesencephalon region (Lysle et al., J. Pharmacol. Exp. Ther. (1996) 277(3), 1533-40; Weber et al., Science (1989) 245, 188-90). Gomez *et al.* investigated the ability of morphine and buprenorphine to influence immune function after central administration (Gomez *et al.,* (2000), *supra*). Acute administration of morphine showed significant decreases in NK cell cytotoxic activity, T lymphocyte proliferative responses to various mitogen and macrophage function, which were associated with high glucocorticoid and catecholamine levels. However, buprenorphine, a partial opioid agonist, did not alter immune function and also failed to increase the peripheral production of plasma glucocorticoids and catecholamines. Morphine has been suggested to induce immunosuppression by interacting with mu₂-opioid receptors, while burprenorphine binds to both mu- and kappa-opioid receptors (Carr et al., J. Pharmacol. Exp. Ther. (1993) 264(3), 1179-86; Kamei et al., Life Sci. (1997) 60(22) 333-7; Pick et al., Brain Res. (1997) 744(1), 41-6).

While the immunological effects of opioids has been demonstrated to have a centrally-mediated mechanism, there is evidence of a coordinated effect in mediating immunosuppression. Two possible pathways that have been implicated in the mediation of the immunomodulatory effects of morphine: the hypothalamic-pituitary-adrenal (HPA) axis and the sympathetic nervous system. Signals from the central nervous system (CNS) to the immune system are relayed primarily through the HPA axis or via sympathetic innervation of lymphoid organs. Thus, opioid action in the HPA axis through hypothalamic efferents or enhanced opioid activity in the PAG could cause an increase in peripheral sympathetic output, either of which could have an effect on NK cell activity (Blalock J. Immunol. (1984) 132(3), 1067-70; Felten, J. Immunol. (1985) 135)2 Suppl), 755a-65s). The activation of the HPA axis results in the downstream production of glucocorticoids which are immunosuppressives (Pruett *et al.*, 1992; Freier and Fuchs, 1994). However, activation of the sympathetic nervous system elicits the release of bioamines which have been demonstrated to suppress the immune system by direct and secondary action on lymphocytes (Fecho et al., J. Pharmacol Exp. Ther. (1996a) 277(2), 633-45). It has been suggested that acute administration of opioids may alter peripheral immune function through the sympathetic nervous system, while chronic administration affects the immune system by activation of the HPA axis (Mellon et al., Brain Res. (1998) 789(1), 56-67).

Investigators have utilized methylnaltrexone, a quaternary derivativeof naltrexone that does not cross the brain blood barrier, to distinguish central from peripheral effects. Methylnaltrexone was shown to antagonize most of the immune system effects of systemic morphine injection when administered intracerebroventricularly, but failed to do so when administered subcutaneously in rats. This suggests that the central opioid receptors play an important role in the immune system alterations by morphine (Lysle et al., Brain Behav. Immun. (1992) 6(2) 179-88; Lysle et al., Int. J. Immunopharmacol. (1995) 17(8), 641-7; Fecho et al., J. Pharmacol. Exp. Ther. (1996b) 276(2), 626-36).

There are also conflicting studies demonstrating that immunological dysfunction is mediated peripherally by opioid receptors on immune cells. Morphine was found to decrease phagocytic activity of macrophages in a concentration-dependent manner, and naltrexone completely blocked the effects of morphine both *in vivo and in vitro* paradigms without affecting phagocytic function (Rajavin et al., Life Sci. (1993) 53, 997-1006). In one *in vitro* study, morphine pellets inhibited the capacity of bone marrow macrophage precursors to develop into viable colonies in response to macrophage colony stimulating factor; and this effect was inhibited by naltrexone (Roy et al., Eur. J. Pharmacol. (1991) 195, 359-363). Also, *in vitro* addition of morphine beta-endorphin to precursor cells of macrophages had similar effect, showing that morphine acted directly on the precursor cells. However, another *in vitro* study contradicted the finding that peripherally-mediated immunosuppression could be reversed by opioid antagonists. Bayer *et al.* observed that morphine inhibited Concanavalin A-induced proliferation of both whole blood and splenic lymphocytes, but this inhibitory effect on the proliferation of lymphocytes was not attenuated by co-incubation with naltrexone (Bayer et al. Immunopharmacology (1992) 23(2), 117-24).

Thomas *et al.* reported that, after *in vitro* exposure to morphine and its metabolites, a number of immunosuppressive effects were observed in immune cells obtained from both laboratory animals and humans (Thomas et al., Pharmacology. (1995) 50, 51-62). Others showed that morphine and kappa-agonists (U50,488H and U69,593) inhibited antibody formation when added to mouse spleen cells *in vitro,* indicating that the effects of opioids may act directly on immune cells (Taub et al., Proc. Natl. Acad. Sci. (1991) 88, 360-4 and Eisenstein et al., J. Pharmacol. Exp. Ther. (1995) 275, 1484-9). Additionally, *in vitro* administration of DAMGO, DPDPE, or U69,593 to splenocyte cultures did not significantly alter the production of nitric oxide by splenocytes. Guan *et al.* found that the kappa-agonist U50,488H inhibited *in vitro* activity of T cell- and macrophage-enriched fractions of normal mouse spleens (Guan et al., Brain Behav Immun. (1994) 8, 229-40). In addition, the suppressive effects of a kappa-agonist were observed on plaque-forming cell antibody response in rats, whether given *in vivo* or *in vitro* (Radulovic et al., Neuroimmunol. (1995) 57, 55-62). It appears that kappa-opioid receptors may be responsible for peripherally-mediated immunosuppression, but a contribution from peripheral mu- or delta-receptors remains to be demonstrated.

Opioids are widely used; they are administered for a variety of medical indications and abused by drug addicts although they have many undesirable side effects. Among the side effects, a clinically important adverse effect is immunosuppression. A close relationship exists between the use of opioids and exacerbated infections, such as AIDS. Additionally, immunosuppression is a dangerous side effect in patients administered opioids following surgery. The mechanism of action of opioid immunosuppressive effects remains controversial. There is compelling evidence that opioids act within the central nervous system to alter immune system activity. However, there is also evidence that opioids have a direct inhibitory effect on immune cells. At the present, the precise mechanism of opioid effects on immunomodulation is not fully understood.

### SUMMARY OF THE INVENTION

The scope of the present invention is limited by the appended claims.

The invention relates to treating mu opioid-induced immune suppression with methylnaltrexone. The invention is based, in part, on the surprising discovery that methylnaltrexone counteracts the immune suppression induced by opioids in immunosuppressed patients receiving opioids. Because of the uncertainty in the mechanism of opioid-induced immunosuppression and the strong evidence of a central nervous system role as described above, it was unpredictable and unexpected that methylnaltrexone would be an effective therapeutic agent for treating immunosuppression caused by opioids. In one aspect of the invention, compositions for treating mu opioids-induced immune suppression in immunosuppressed patients receiving an opioid are provided for administering methylnaltrexone to a patient in an effective amount to treat the mu opioid-induced immune suppression. Said composition is

a pharmaceutical composition comprising methylnaltrexone for treating mu opioid agonist induced immune suppression in a patient, wherein the pharmaceutical composition is to be administered subcutaneously at a dosage ranging from 0.001 to 5 mg/kg body weight of the patient, or enterally, intravenously, orally, transdermally, transmucosally, or rectally.

The opioid is a mu opioid agonist. In some embodiments, more than one opioid agonist is administered to the patient, including combinations of mu agonists, and combinations of mu and kappa agonists.

Methylnaltrexone is a mu opioid antagonist, and is a quaternary derivative of noroxymorphone.

In some embodiments of the invention, compositions for use in treating mu opioid-induced immune suppression in patients receiving an opioid are provided, wherein methylnaltrexone and at least one pharmaceutical agent that is not an opioid or opioid antagonist are administered to the patient. Suitable pharmaceutical agents include antiviral agents, antiretroviral agents, anti-infective agents, anticancer agents (including chemotherapeutic agents), CCR5 downregulating agents, and hematopoetic stimulating agents. Suitable antiretroviral agents include, but are not limited to, protease inhibitors, reverse transcriptase inhibitors (including non-nucleoside inhibitors), integrase inhibitors, nucleoside analogs, and nucleotide analogs.

In some embodiments, the patients treatable by the compositions of the invention are cancer patients. Other patients treatable by the compositions of the invention have been exposed to radiation. Some of the patients treatable by the compositions and methods of the invention are patients who have been exposed to one or more chemotherapeutic agents.

In some embodiments, the patients treatable by the compositions of the invention are infected with HIV, Hepatitis C or Cytomegalovirus. In some of these embodiments, the patients have AIDS. Still other patients treatable by the compositions of the invention are patients with autoimmune disorders. Other immunosuppressed patients treatable by the compositions and uses of the invention also include patients with secondary opportunistic infections.

In some embodiments, the methylnaltrexone is administered to the patient in an amount effective to inhibit an opioid-induced increase in the patient's viral load. In other embodiments, the methylnaltrexone is administered in an amount effective to inhibit an opioid-induced increase in the patient's CCR5 level or CCR5 receptor expression on cells capable of expressing CCR5. Such cells include primary T cells, monocytes, macrophages and glial cells. In still other embodiments, the methylnaltrexone is administered in an amount effective to inhibit an opioid-induced decrease in the patient's amount of CD4 positive T cells.

In some embodiments of the invention, compositions for treating mu opioid-induced immune suppression in patients receiving an opioid are provided wherein the patients are administered methylnaltrexone in an effective amount to treat the mu opioid-induced immune suppression and parameters indicative of the level of immunosuppression of the patient are monitored. In some embodiments, the patient's viral load is monitored. In other embodiments, the patient's CCR5 levels are monitored. CCR5 levels are monitored on patient cells capable of CCR5 expression. These cells include, primary T-cells, monocytes, macrophages, and glial cells. In yet other embodiments, the patient's amount of CD4 positive cells, such as monocytes, macrophages, and T cells, are monitored.

In some embodiments of the invention, compositions are provided to treat chronic opioid users. In some embodiments, the opioid is methadone, morphine, or heroin. In other embodiments, the opioid is a mixed agonist such as butorphanol. In some embodiments of the invention, the patients are opioid addicts. In other embodiments, the patients are administered more than one opioid, for example, morphine and heroin or methadone and heroin. These include patients who are opioid abusers and receive many opioids concurrently.

In some embodiments of the invention, the methylnaltrexone is administered in a formulation comprising methylnaltrexone and an opioid. The formulation may be an oral, liquid, suspension, or other formulation, known in the art, such as a lyophilized powder or a time-release formula.

The methylnaltrexone may be administered using any conventional mode of administration, known to those of skill in the art. The methylnaltrexone may be administered enterally. Modes of administration include, but are not limited to, intravenous, subcutaneous at a dosage ranging from 0.001 to 5mg/kg body weight, oral, transdermal, transmucosal, topical, and rectal administration. Additionally, the methylnaltrexone may be administered as an enterically coated tablet or capsule. In some embodiments, the methylnaltrexone is administered by a slow infusion method or by a time-release method.

When the methylnaltrexone is administered intravenously or subcutaneously, the dosage may range from 0.001 to 5 mg/kg body weight of the patient. In some embodiments, the dosage may range from 0.05 to 0.5 mg/kg body weight of the patient. For subcutaneous administration, it is preferred to administer a volume of 0.5 to 1.5 cc, to the patient to avoid pain. When the methylnaltrexone is administered orally, the dosage may range from 1 to 80 mg/kg body weight of the patient. In some embodiments, the oral dose may range from 2 to 20 mg/kg body weight of the patient. The dosage depends on the formulation used, for example, oral doses with enteric coatings are typically administered in amounts lower than oral doses that are not enterically coated. Suitable dosage units can be determined by those of skill in the art.

In some embodiments of the invention, the patient's plasma level of methylnaltrexone does not exceed 1000 ng/ml. The methylnaltrexone may be administered in an effective amount such that the patient's plasma level of methylnaltrexone does not exceed 750, 500, 400, 300, 250, 200, 150, 100, 50, or even 20 ng/ml. Patient drug plasma levels may be measured using routine HPLC methods known to those of skill in the art.

In yet another aspect of the invention, formulations comprising an opioid, methylnaltrexone, and a pharmaceutical agent that is not an opioid or opioid antagonist are provided. The opioids and methylnaltrexone are as described above. The pharmaceutical agents include antiviral agents, antiretroviral agents, antiinfective agents, anticancer agents, CCR5 downregulating agents, and hematopoetic agents. The formulations may be prepared using standard formulation methods known to those of skill in the art.

Another aspect of the present invention is a composition for preventing or inhibiting infection of CCR5 positive cells by macrophage-tropic HIV-1 in a patient receiving at least one opioid comprising administering to the patient methylnaltrexone in an effective amount to prevent or treat infection of cells by macrophage-tropic HIV-1. Cells amenable to such treatment include CD4 positive cells, in particular, CD4 positive cells that express or are capable of expressing CCR5.

In another aspect of the invention, compositions for treating mu opioid-induced immune suppression in immunosuppressed patients receiving an opioid are provided comprising administering to the patients methylnaltrexone in an effective amount to treat the mu opioid-induced immune suppression. The preferred embodiments are described above, as if specifically recited herein.

### DETAILED DESCRIPTION

The present invention relates to compositions for use in treating mu opioid-induced immune suppression in immunosuppressed patients receiving an opioid comprising administering methylnaltrexone in an effective amount to the patient to treat the mu opioid-induced immune suppression. The invention is based, in part, on Applicants' unpredictable discovery that methylnaltrexone is useful to treat mu opioid-induced immune suppression.

The patients treatable by the compositions of the invention are patients who are receiving opioids or will receive opioids. Immunosuppressed patients have been exposed to one or more events that measurably weakens their immune system, such as opioid administration, a viral infection, radiation, administration of a pharmaceutical agent (such as a chemotherapeutic agents) which causes immune suppression. "Immune suppression" and "immunosuppression" are used interchangeably herein.

The patients treatable by the compositions of the invention include patients infected with HIV, some of which have AIDS. These patients include patients infected with HIV-1, including, HIV-1 strains such as macrophage-tropic HIV-1 strains that utilize CCR5 as a co-receptor for entry into CCR5 positive host cells. Other patients treatable by the compositions of the invention include patients with other viral infections, such as Hepatitis C, Cytomegalovirus, kidney failure, liver failure, malnutrition, alcoholism, hepatitis, protein-losing enteropathy, autoimmune diseases, secondary opportunistic infections, such as caused by *Pneumocystis carinii,* Toxoplasma, Mycobacterium, Cryptocuccus, Candida, cancer, and primary malignancies of the immune system (including lymphomas, leukemias, and multiple myelomas), post-splenectomy patients, and patients with other immunosuppressing disorders known to those of skill in the art. Other patients treatable by the compositions of the invention have been or will be exposed to radiation, for example, as part of an anticancer therapy, or from receiving x-rays.

Some patients treatable by the compositions of the invention are chronic opioid users. As used herein, a "chronic opioid user" is a patient which has been administered opioids for 14 days or more. Some chronic opioid users are opioid addicts. Many chronic opioid users are administered methadone as part of a chronic opioid user maintenance program. Other chronic opioid users include terminally ill patients who are administered morphine for pain relief including palliative care. Acute opioid users, i.e., patients who have been administered opioids for fewer than 14 days, who are immunosuppressed are also treatable by the compositions of the invention.

Methylnaltrexone is administered in effective amounts. An "effective amount" means that amount necessary to delay the onset of, inhibit the progression of, halt altogether the onset of, or halt altogether the progression of mu opioid-induced immune suppression, or any amount with a measurable reversal of immune suppression. Accordingly, an effective amount may not necessarily have a clinical impact. An effective amount may be determined directly or indirectly.

Direct uses generally include measuring the patient's degree of immune suppression. Amounts of immune suppression are typically determined by monitoring immune system cell counts, immune system receptor levels, immune system activation, or any other immune system indicators, as are commonly known in the art. These indicators include CCR5 levels, CCR5 receptor expression or modulation, lymphocyte activation, viral load, CD4 positive T cell count, specific antibodies, natural killer cell count, lymphocytes, helper cells, and cytokine release.

Indirect methods of determining an effective amount utilize statistical analysis based on the therapeutic responses to a plurality of dose and administration regimens administered to a plurality of subjects. For example, is the comparison of dose and/or administration test values in test and control patients enrolled in a clinical trial, such as the trial described in the Examples. The responses of the test versus control groups may be compared and dose and/or administration regimen at which there is a statistically significant reduction in the likelihood of immunosuppression may be determined. Other direct and indirect methods will be known to those of ordinary skill in the art and may be employed to assess an effective amount.

CCR5 levels, CCR5 receptor expression, and CCR5 modulation are typically monitored by obtaining patient samples of cells that express CCR5, including, but not limited to, primary T-cells, monocytes, macrophages, and glial cells by determining cell surface CCR5 expression or modulation using immunoassays such as FACS analysis or other CCR5 receptor assays as are known in the art such as those described in Olson, W.C., et al., J. Virology, 73(5), 4145-4155 and Methods in Molecular Biology, 138, Ed. by Amanda E.I. Proudfoot et al., Humana Press, Totowa, NJ, 2000. Monoclonal antibodies to CCR5 useful in such assays are described in WO/0035409A2. Another monoclonal anti-CCR5 antibody, Mab 2D7, is available from Pharminogen (San Diego, CA).

Lymphocyte activation such as T cell activation is measurable using methods known to those of skill in the art, such as by mitogen assays, antigen-specific lymphocyte activation assays, cytokine assays, and the like (Current Protocols in Immunology, Vols. 1-4, Wiley).

Viral load is measurable using methods known to those of skill in the art. A commercial assay, Amplicor HIV-1 Monitor^{™} test, is available from Roche Diagnostics Corp. CD4 positive cell counts are measurable using methods known to those of skill in the art. Similarly, natural killer cell counts, lymphocyte counts, helper cell counts are also measurable using methods known to those of skill in the art (Current Protocols in Immunology, Vols. 1-4, John Wiley & Sons, Publishers).

An effective amount in connection with treating infectious disease is that amount necessary to delay the onset of, inhibit the progression of, or halt altogether the onset or progression of the infection. In particular embodiments, the infection is a retroviral infection, and most particularly an HIV infection. In general, an effective amount will be that amount necessary to inhibit the symptoms or physiological (e.g., immunological or viral) characteristics of the viral infection, any of which otherwise would have occurred in a subject experiencing a viral infection absent the treatment of the invention. Several parameters may be used to assess reduction of viral infection, including inhibited viral replication, a lessened decrease of CD4 (positive) cells such as T cells (CD4+ T cell) counts, a stabilization of CD4+ T cell count or even an increased CD4+ T cell count, and/or an inhibited increase of viral load or even a decreased viral load, for example, as compared to pretreatment patient parameters, untreated patients or, in the case of treatment with cocktails, patients having a viral infection treated with antiviral agents alone (i.e. without methylnaltrexone). These parameters can be monitored using standard diagnostic procedures including ELISA, polymerase chain reaction (PCR and RT-PCR), and flow cytometry.

In one embodiment of the present invention, the methylnaltrexone is effective in increasing CD4+ T cell counts by at least 5%, at least 10%, or even at least 25% or greater, compared to the pretreatment CD4+ T cell count of the patient (which may be determined directly, or indirectly using statistical methods).

The methylnaltrexone useful in the present invention is peripherally acting. "Peripheral" as used herein, refers to the inability to cross the blood-brain barrier in an effective amount to inhibit the central effects of opioids. Methylnaltrexone does not effectively inhibit the analgesic effects of opioids when administered peripherally. Methylnaltrexone is a mu opioid antagonist. Antagonists are classified by their ability to antagonize one receptor an order of magnitude more effectively than another receptor. For example, the opioid antagonist naloxone acts as a competitive antagonist at all opioid receptors, but is approximately ten times more effective at mu receptors than at kappa receptors and is therefore classified as a mu opioid antagonist. Generally, the mu receptor is associated with pain relief, drug and chemical relief, and chemical dependence (e.g., drug addiction and alcoholism).

Similarly, the compositions of the invention encompass treating patients who are administered mu opioid agonists such as morphine, methadone, heroin, codeine, meperidine, fentidine, fentanil, sufentanil, alfentanil. Opioid agonists are classified by their ability to agonize one type of receptor an order of magnitude more effectively than another. For example, the relative affinity of morphine for the mu receptor is 200 times greater than for the kappa receptor, and is therefore classified as a mu opioid agonist. Some opioid agonists may act as agonists towards one receptor and antagonists toward another receptor and are classified as agonist/antagonists, (also known as mixed or partial agonists). "Agonist/antagonist," "partial agonist," and "mixed agonist" are used interchangeably herein. These opioids include pentazocine, butorphanol, nalorphine, nalbufine, buprenorphine, bremazocine, and bezocine. Many of the agonist/antagonist group of opioids are agonists at the kappa and sigma receptors and antagonists at mu receptors.

Methylnaltrexone is a quaternary derivative of noroxymorphone, described first by Goldberg, et al., in U.S. Patent No. 4,176,186. Methylnaltrexone is also described in U.S. Patent Nos. 4,719,215; 4,861,781; 5,102,887; 5,972,954; 6,274,591; and PCT publication Nos. WO 99/22737 and WO 98/25613.

Methylnaltrexone is provided as a white crystalline powder freely soluble in water. Its melting point is 254-256 °C. The compound as provided is 99.4% pure by reverse phase HPLC, and contains less than 0.011 % unquaternized naltrexone by the same method. Methylnaltrexone is also identified as N-methyl-naltrexone bromide, methylnaltrexone, MNTX, SC-37359, MRZ-2663-BR), and N-cyclopropylmethylnoroxymorphine-methobromide. Methylnaltrexone is available in a powder form from Mallinckrodt Pharmaceuticals, St. Louis, MO. Methylnaltrexone can be prepared as a sterile solution at a concentration of 5 mg/ml. Methylnaltrexone can also be administered as an oral agent in a capsule or tablet or in an oral solution.

In some embodiments of the invention, methylnaltrexone is administered in a formulation comprising the methylnaltrexone and the opioid. These formulations may be oral, such as the formulations described in U.S. Patent Nos. 6,277,384; 6,261,599; 5,958,452 and PCT publication No. WO 98/25613.

The compositions for treating mu opioid-induced immune suppression may also comprise administering methylnaltrexone and at least one pharmaceutical agent that is not an opioid or opioid antagonist to a patient suffering from mu opioid-induced immune suppression. Pharmaceutical agents include, but are not limited to, antiviral agents, antiretroviral agents, anti-infective agents, anticancer agents, CCR5 down-regulating agents, and hematopoetic stimulating agents.

Antiviral agents include nucleoside analogs, nonnucleoside reverse transcriptase inhibitors, nucleoside reverse transcriptase inhibitors, protease inhibitors, integrase inhibitors, including the following: Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscarnet Sodium; Fosfonet Sodium; Ganciclovir; Ganciclovir Sodium; Idoxuridine; Indinavir; Kethoxal; Lamivudine; Lobucavir; Lopinovir; Memotine Hydrochloride; Methisazone; Nelfinavir; Nevirapine; Penciclovir; Pirodavir; Ribavirin; Rimantadine Hydrochloride; Ritonavir; Saquinavir Mesylate; Somantadine Hydrochloride; Sorivudine; Statolon; Stavudine; Tenofovir; Tilorone Hydrochloride; Trifluridine; Valacyclovir Hydrochloride; Vidarabine; Vidarabine Phosphate; Vidarabine Sodium Phosphate; Viroxime; Zalcitabine; Zerit; Zidovudine (AZT); and Zinviroxime.

Anti-infective agents include Difloxacin Hydrochloride; Lauryl Isoquinolinium Bromide; Moxalactam Disodium; Ornidazole; Pentisomicin; Sarafloxacin Hydrochloride; Protease inhibitors of HIV and other retroviruses; Integrase Inhibitors of HIV and other retroviruses; Cefaclor (Ceclor); Acyclovir (Zovirax); Norfloxacin (Noroxin); Cefoxitin (Mefoxin); Cefuroxime axetil (Ceftin); Ciprofloxacin (Cipro); Aminacrine Hydrochloride; Benzethonium Chloride : Bithionolate Sodium; Bromchlorenone; Carbamide Peroxide; Cetalkonium Chloride; Cetylpyridinium Chloride : Chlorhexidine Hydrochloride; Clioquinol; Domiphen Bromide; Fenticlor; Fludazonium Chloride; Fuchsin, Basic; Furazolidone; Gentian Violet; Halquinols; Hexachlorophene : Hydrogen Peroxide; Ichthammol; Imidecyl Iodine; Iodine; Isopropyl Alcohol; Mafenide Acetate; Meralein Sodium; Mercufenol Chloride; Mercury, Ammoniated; Methylbenzethonium Chloride; Nitrofurazone; Nitromersol; Octenidine Hydrochloride; Oxychlorosene; Oxychlorosene Sodium; Parachlorophenol, Camphorated; Potassium Permanganate; Povidone-Iodine; Sepazonium Chloride; Silver Nitrate; Sulfadiazine, Silver; Symclosene; Thimerfonate Sodium; Thimerosal: Troclosene Potassium.

Anti-cancer agents (chemotherapeutic agents), include, anti-cancer drugs. Anti-cancer drugs are well known and include: Acivicin; Aclarubicin; Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflomithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1 ; Interferon Alfa-n3; Interferon Beta-I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talisomycin; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride.

Other anti-cancer drugs include: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; fmasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone +pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene dichloride; topotecan; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; zinostatin stimalamer.

Supplementary potentiating agents likewise are well characterized and include: Tricyclic anti-depressant drugs (e.g., imipramine, desipramine, amitryptyline, clomipramine, trimipramine, doxepin, nortriptyline, protriptyline, amoxapine and maprotiline); non-tricyclic anti-depressant drugs (e.g., sertraline, trazodone and citalopram); Ca²⁺ antagonists (e.g., verapamil, nifedipine, nitrendipine and caroverine); Calmodulin inhibitors (e.g., prenylamine, trifluoroperazine and clomipramine); Amphotericin B; Triparanol analogues (e.g., tamoxifen); antiarrhythmic drugs (e.g., quinidine); antihypertensive drugs (e.g., reserpine); Thiol depleters (e.g., buthionine and sulfoximine) and Multiple Drug Resistance reducing agents such as verapamil, cyclosporin A and Cremaphor EL.

Hematopoetic stimulating agents include G-CSF, M-CSF, erythropoietin (EPO), thrombopoietin (TPO), the interleukins (IL-1, IL-2, IL-3, IL-4, IL-5, IL-6, IL-7, IL-8, IL-9, IL-10, IL-11, IL-12, IL-13, IL-14, IL-15), granulocyte-macrophage colony-stimulating factor (GM-CSF) and stem cell factor and leukemia inhibitory factor (LIF).

In one aspect of the invention, a pharmaceutical composition comprising methylnaltrexone, one or more pharmaceutical agents, and pharmaceutically acceptable excipients are prepared for administration to patients in need of such treatment. One of ordinary skill in the art is familiar with a variety of pharmaceutical agents which are used in the medical arts to treat patients. These include, antiviral agents used in HIV cocktails. One embodiment of the composition comprises methylnaltrexone, efavirenz (Dupont/Merck), and AZT. In another embodiment of the composition the composition comprises methylnaltrexone and COMBIVIR (Lamivuaine and Zidovudine; Glaxo Wellcome).

A variety of administration routes are available. The particular mode selected will depend, of course, upon the particular combination of drugs selected, the severity of the immunosuppression being treated, or prevented, the condition of the patient, and the dosage required for therapeutic efficacy. The compositions of this invention, generally speaking, are used for any mode of administration that is medically acceptable, meaning any mode that produces effective levels of the active compounds without causing clinically unacceptable adverse effects. Such modes of administration include oral, rectal, topical, transdermal, sublingual or intramuscular, infusion, intravenous, pulmonary, intramuscular, intracavity, as an aerosol, aural (e.g., via eardrops), intranasal, inhalation, or subcutaneous at a dosage ranging from 0.001 to 5mg/kg body weight of patient. Direct injection could also be preferred for local delivery. Oral or subcutaneous administration may be preferred for prophylactic or long-term treatment because of the convenience of the patient as well as the dosing schedule.

Generally, oral doses of methylnaltrexone will be from 1 to 80 mg/kg body weight per day. It is expected that oral doses in the range from 2 to 20 mg/kg body weight will yield the desired results. Generally, administration, including intravenous and subcutaneous administration, will be from 0.001 to 5 mg/kg body weight. It is expected that doses ranging from 0.05 to 0.5 mg/kg body weight will yield the desired results. Dosage may be adjusted appropriately to achieve desired drug levels, local or systemic, depending on the mode of administration. For example, it is expected that the dosage for oral administration of methylnaltrexone in an enterically-coated formulation would be from 10 to 30% of the non-coated oral dose. In the event that the response in a patient is insufficient of such doses, even higher doses (or effectively higher dosage by a different, more localized delivery route) may be employed to the extent that the patient tolerance permits. Multiple doses per day are contemplated to achieve appropriate systemic levels of compounds. Appropriate system levels can be determined by, for example, measurement of the patient's plasma level of the drug.

When administered, the formulations of the invention are applied in pharmaceutically acceptable amounts and in pharmaceutically acceptable compositions. Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulphuric, nitric, phosphoric, maleic, acetic, salicylic, p-toluenesulfonic, tartaric, citric, methanesulfonic, formic, succinic, naphthalene-2-sulfonic, pamoic, 3-hydroxy-2-naphthalenecarboxylic, and benzene sulfonic. Also, pharmaceutically acceptable salts can be prepared as alkaline metal or alkaline earth salts, such as sodium, ammonium, magnesium, potassium or calcium salts of the carboxylic acid group.

Suitable buffering agents include: acetic acid and salts thereof (1-2% W/V); citric acid and salts thereof (1-3% W/V); boric acid and salts thereof (0.5-2.5% W/V); and phosphoric acid and salts thereof (0.8-2% W/V).

Suitable preservatives include benzalkonium chloride (0.003-0.03% W/V); chlorobutanol (0.3-0.9% W/V); parabens (0.01-0.25% W/V) and thimerosal (0.004-0.02% W/V).

The compositions may conveniently be presented in unit dosage form and may be prepared by any of the methods well known in the art of pharmacy. All methods include the step of bringing the compounds of the invention into association with a carrier which constitutes one or more accessory ingredients. In general, the compositions are prepared by uniformly and intimately bringing the compounds of the invention into association with a liquid carrier, a finely divided solid carrier, or both, and then, if necessary, shaping the product.

Other delivery systems can include time-release, delayed release or sustained release delivery systems. Such systems can avoid repeated administrations of the compounds of the invention, increasing convenience to the patient and the physician. Many types of release delivery systems are available and known to those of ordinary skill in the art. They include polymer based systems such as polylactic and polyglycolic acid, polyanhydrides and polycaprolactone, nonpolymer systems that are lipids including sterols such as cholesterol, liposomes; phoshpholipids; hydrogel release systems; silastic systems; peptide based system; implants and the like. Specific examples include, but are not limited to: (a) erosional systems in which the polysaccharide is contained in a form within a matrix, found in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hard wired delivery systems can be used, some of which are adapted for implantation. Alternatively, enteric coatings may be employed. Suitable coatings are described in PCT publication No. WO 98/25613 and U.S. Patent No. 6,274,591.

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30 to 60 days. The implant may be positioned at the site of injury. Long-term sustained release implants are well-known to those of ordinary skill in the art and include some of the release systems described above.

### EXAMPLES

The following Examples are intended to illustrate aspects of the invention The Examples describe a clinical trial to study the safety and efficacy of a peripheral opioid antagonist to treat opioid-induced immune suppression. More specifically, this trial evaluates the effect of multiple-dosed subcutaneous methylnaltrexone in a target population of HIV positive patients enrolled in methadone maintenance programs. Pharmacokinetic data of subcutaneous methylnaltrexone are also obtained.

### Example 1: Methylnaltrexone dose

The dose of methadone is 30 to 200 mg/day and the dose of methylnaltrexone is approximately 0.01 to 0.3 mg/kg (0.7 to 21 mg in a 70 kg subject or lower), every eight hours for two consecutive days. The doses for each subject are calculated based on the subject's weight at the start of the trial. The drug administration is done under the supervision of the investigator.

In a previous controlled volunteer study (Yuan et al., Clin. Pharmacol. Ther. (1996) 59, 496-475), intravenous methylnaltrexone 0.45 mg/kg was not associated with any overt side effects. Because chronic opioid users have an increased sensitivity to methylnaltrexone, it is believed that the therapeutic dose of methylnaltrexone will not exceed 0.3 mg/kg. Because the half-life of the intravenous compound is less than two hours, drug accumulation in this study should be minimal (Yuan et al., (1996), *supra*). The extent of drug accumulation at steady state is determined by pharmacokinetic analysis of plasma samples drawn during the study.

### Example 2: Study Subjects

The subjects are selected using the following criteria. The inclusion criteria is as follows: First, subjects must be male or non-pregnant female volunteers. Second, subjects must be 18 to 65 years of age inclusive. Third, subjects must have no significant active disease states (as described below).

The exclusion criteria is as follows: Patients with a history or current evidence of significant (outside normal laboratory limits) cardiovascular, respiratory, endocrine, renal, hepatic, hematological or psychiatric disease will be excluded. Subjects who currently use of OTC (over the counter) or prescription medications, or who use of medications within seven days prior to study initiation that might confound the study of methylnaltrexone (e.g., opioids, laxatives) will be excluded. Subject with any laboratory findings which are outside normal limits or physical examination findings which are abnormal that might confound the study of methylnaltrexone is excluded. Subjects with known hypersensitivity to naltrexone or morphine, or lactulose, galactose or lactose intolerance will be excluded. Illicit drug users, subject who have participated in any investigational new drug study in the previous 30 days, and subjects who have not given consent to participate or not signed a consent form will also be excluded.

Subjects who do not complete the study for any reason not related to the study drug are replaced with an alternate volunteer. Subjects are withdrawn for any of the following reasons: (1) The subject requests withdrawal; (2) The investigator requests the subject's withdrawal for reasons not related to the drug; (3) A significant adverse event or alteration in a clinical laboratory value necessitates withdrawal in the investigator's opinion. Subjects who are withdrawn from the study are asked to return for a safety visit. If a subject still shows signs of a drug effect, the subject is kept in the Clinical Research Center (CRC) for further observation. Data from all subjects is included in the final study analysis.

Subjects must agree to discontinue their medications at the screening or at least one week before receiving the study medication. Subjects must not take any other medication for the duration of the study period. Subjects must abstain from the use of alcohol during the study period. Twelve (12) subjects who meet the above criteria are enrolled in the study.

### Example 3: Research Protocol

The study is designed to evaluate the safety and effects of methylnaltrexone in normal volunteers. One of skill in the art will recognize that the study may be designed to evaluate the effects of methylnaltrexone in healthy volunteers (as a control) and opioid users (such as subjects enrolled in a methadone maintenance program). Hemodynamic parameters are collected. Each subject receives multiple intravenous doses of methylnaltrexone in a study period of approximately 48 hours. The duration of the study, including screening and safety visit will be approximately two weeks.

Each subject receives intravenous methylnaltrexone in a dose ranging from 0.05 to 0.15 mg/kg, every eight hours for two consecutive days. 10cc of whole blood is harvested on five occasions and CCR5 receptor expression on cells. Subjects are admitted to the Clinical Research Center for approximately 56 hours. During this period, they are monitored for potential adverse effects to methylnaltrexone (including potential withdrawal), complete subjective questionnaires, and are monitored for pharmacological response to the study drug. Adverse reactions are recorded and followed for severity and outcome.

### Example 4: Study Procedures

The subject candidates are screened as follows: Candidates are given a health questionnaire covering significant medical problems. If the volunteer initially meets the criteria, the study is explained to them and a signed consent form is obtained. After a careful history and physical exam, vital signs are recorded. A 12 lead, supine, resting electrocardiogram is obtained. Laboratory studies are obtained at the screening, including, hematology (CBC with differential and platelet count), a comprehensive panel (sodium, potassium, chloride, BUN, creatinine, glucose, calcium, total protein, albumin, alkaline phosphatase, SGOT, and total bilirubin), uine analysis, and tests for illicit drug use. Females of child-bearing potential need a pregnancy test prior to drug administration. The subjects are asked to have a standard meal consisting of a serving of boiled rice or white bread, a steak or hamburger patty or chicken, and water *ad libitum* before 8 PM the day before the drug trial. Subjects are admitted on the morning of the study after fasting from midnight.

A urine sample is obtained before drug administration. The subject's weight is recorded. Heart rate (HR), and blood pressure (BP) are obtained after 10 minutes of quiet rest. An intravenous catheter is placed into the subject for drug blood drawing. An infusion of normal saline will be given as needed. Approximately 10 ml of blood is drawn at the time of screening The subjects complete a written subjective questionnaire.

At time 0 and 15 minutes after the first, third and final dose of the study drug, an additional 10 cc of blood is withdrawn from the catheter. At time 0, the subject is given methylnaltrexone 0.15 mg/kg subcutaneously every 8 hours for 48 consecutive hours (6 doses). During the 76 hours beginning from time 0, the following is monitored: (1) Vital signs (BP, HR) at time 5, 15, 30, 45, 60 minutes and 2, 3, 4 and 6 hours, for the first and the last methylnaltrexone administration. (2) Venous blood samples (10 ml each) are drawn for measurement of plasma drug levels at time 0, and 15 minutes after the first, third and final methylnaltrexone administrations. (3) For the remaining methylnaltrexone administrations, vital signs and venous blood samples (5 ml) are treated immediately before and 5 minutes after the drug administrations. (4) All adverse experiences are recorded on the case report form with special notes made of time of onset and resolution, severity, and the investigator's opinion of the relationship of any adverse experiences to the drug. An independent group reviews all such events. (5) Opioid subjective effects are obtained just before the first, third and final methylnaltrexone doses using a modified adjective checklist, reflecting opioid medication effects (Yuan et al., 1998d). This list consists of 12 items: "flushing," "stimulated," "numb," "drunken," "difficulty in concentrating," "drowsy (sleepy)," "coasting or spaced out," "turning of stomach," "skin itch," "dry mouth," "dizzy," and "nauseous". Subjects are instructed to rate each of these items on a 5-point scale from 0 ("not at all") to 4 ("extremely"). After each test, the ratings for the 12 individual items are summed to give a total subjective symptom score.

### Example 5: Handling of Samples for Pharmacokinetics Analysis

Blood is drawn from the arm catheter used for methylnaltrexone injection into EDTA Vacutainers prelabeled with the study number, subject number and initials, dose number, date, time of sample, at the times indicated above. Samples are centrifuged and frozen at -80 ° C. All samples for each subject are stored collectively in a sealable polyethylene bag, labeled with study information using an indelible black marker.

Pharmacokinetic parameters (Cₘₐₓ, peak free plasma concentration; Tₘₐₓ, time to peak plasma concentration; AUC, area under the plasma concentration-time curve from 0 to 6 hr; V_{d}β, apparent volume of distribution during β phase; t_{1/2}, β half-life; CL, total body clearance; Fᵤ, percentage of dose excreted unchanged in urine) are tabulated. The pharmacokinetic analysis for methylnaltrexone is obtained using a SAAM II numerical model for two compartments (Yuan *et al.*, (1996), *supra*) or an equivalent model.

### Example 6: CCR5 Expression Analysis

Flow cytometry is used to detect CCR5 protein expression using monoclonal antibodies to CCR5 such as Mab2D7. CD4+ lymphocytes from peripheral blood mononuclear cells (PBMC) are isolated and incubated with 5 µg of antibody per ml for 20 minutes at 4 °C in Dulbecco's PBS containing 0.1% sodium azide. The cells are spun, washed and incubated with phycoerythrin (PE)-labeled goat anti-mouse IgG (Caltag, Burlingame, CA) diluted 1:100 under the same conditions as the first antibody incubation. CCR5 expression is measured by flow cytometry (Olson, W.C. et al., J. Virology 73, 4145-4155).

The number of CCR5 binding sites on lymphocytes is calculated with each patient serving as his/her own control. Initial evaluation will be by paired t tests and Wilcoxon signed rank test. In all cases P < 0.05 is considered significant.

### Example 7: Animal Study

Three groups of mice (ten mice per group) were used to investigate the effect of a peripheral opioid antagonist on opioid-induced immune system changes. Group 1 was treated with two placebos, Group 2 was treated with a placebo and morphine, and Group 3 was treated with methylnaltrexone and morphine. The number of natural killer cells present after treatment was assayed. Group 1 served as the control group. Group 2 showed a significant decrease in the number of natural killer cells compared to Group 1. Group 3 showed comparable number of natural killer cells to Group 1, demonstrating the inhibition of the immunosuppressive effects of morphine by methylnaltrexone.

## Claims

1. A pharmaceutical composition comprising methylnaltrexone for treating mu opioid agonist induced immune suppression in a patient, wherein the pharmaceutical composition is to be administered subcutaneously at a dosage ranging from 0.001 to 5 mg/kg body weight of the patient, or enterally, intravenously, orally, transdermally, transmucosally, or rectally.

2. A composition as claimed in claim 1, wherein said patient receives at least one further pharmaceutical agent that is not an opioid or opioid antagonist.

3. A composition as claimed in claim 2, wherein the pharmaceutical agent is an antiviral agent.

4. A composition as claimed in claim 3, wherein the antiviral agent is an antiretroviral agent.

5. A composition as claimed in claim 3, wherein the antiviral agent is a protease inhibitor.

6. A composition as claimed in claim 3, wherein the antiviral agent comprises a nucleoside analog or nucleotide analog.

7. A composition as claimed in claim 2, wherein the pharmaceutical agent is selected from the group consisting of an anti-infective agent, an anticancer agent or a hematopoetic stimulating agent.

8. A composition as claimed in any of claims 1 to 7, wherein the patient is immunosuppressed.

9. A composition as claimed in claim 1 to be administered intravenously at a dosage ranging from 0.001 to 5mg/kg body weight of the patient.

10. A composition as claimed in claim 1 to be administered intravenously at a dosage ranging from 0.05 to 0.5 mg/kg body weight of the patient.

11. A composition as claimed in claim 1 to be administered subcutaneously at a dosage ranging from 0.05 to 0.5 mg/kg body weight of the patient.

12. A composition as claimed in claim 1 to be administered orally at a dosage ranging from 1 to 80 mg/kg body weight of the patient.

13. A composition as claimed in claim 12 to be administered orally at a dosage ranging from 2 to 20 mg/kg body weight of the patient.

14. A composition as claimed in any one of claims 1, 12 or 13, wherein the pharmaceutical composition is in the form of an enterically coated tablet or capsule.

15. A composition as claimed in claim 1, wherein the pharmaceutical composition comprises methylnaltrexone and the opioid.

16. A composition as claimed in claim 1, wherein the pharmaceutical composition comprises at least one further pharmaceutical agent.

17. A composition as claimed in any of claims 1 to 16, wherein the patient is infected with HIV.

18. A composition as claimed in claim 17, wherein the patient has AIDS.

19. A composition as claimed in claims 1, 15-18, wherein the pharmaceutical composition comprises methylnaltrexone in an amount effective to inhibit an opioid-induced increase in the patient's viral load.

20. A composition as claimed in claim 19, wherein the patient's viral load is monitored.

21. A composition as claimed in claims 1, 15-18, wherein the pharmaceutical composition comprises methylnaltrexone in an amount effective to inhibit an opioid-induced increase in the patient's CCR5 levels.

22. A composition as claimed in claim 21, wherein the patient's CCR5 levels are monitored.

23. A composition as claimed in claims 1, 15-18, wherein the pharmaceutical composition comprises methylnaltrexone in an amount effective to inhibit an opioid-induced decrease in the patient's amount of CD4 positive T cells.

24. A composition as claimed in claim 23, wherein the patient's amount of CD4 positive T cells is monitored.

25. A composition as claimed in any one of claims 1 to 24, wherein the patient has been exposed to radiation.

26. A composition as claimed in any of claims 1 to 25, wherein the patient is a chronic opioid user.

27. A composition as claimed in claim 26, wherein the opioid is methadone or morphine.

28. A composition as claimed in claim 27, wherein the patient is an opioid addict.

29. A composition as claimed in claim 1, wherein the patient's plasma level of methylnaltrexone does not exceed 1000 ng/ml, 500ng/ml, 250 ng/ml, 150 ng/ml, 100ng/ml, or 50ng/ml.

30. A composition as claimed in claim 1 or 29, wherein the pharmaceutical composition is to be administered by a slow infusion method.

31. A composition as claimed in claim 30, wherein the pharmaceutical composition comprises methylnaltrexone and the opioid.

32. Methylnaltrexone for treating mu opioid agonist induced immune suppression, wherein infection by macrophage-tropic HIV-1 of CCR5 positive cells of the patient is to be inhibited.

33. Methylnaltrexone for treating mu opioid agonist induced immune suppression in a patient infected with HIV, Hepatitis C or Cytomegalovirus.

34. Use of a pharmaceutical composition as defined in any one of claims 1 to 33, for the manufacture of a medicament for the treatment of mu opioid agonist induced immune suppression in a patient, wherein the pharmaceutical composition is to be administered subcutaneously at a dosage ranging from 0.001 to 5 mg/kg body weight of the patient, or enterally, intravenously, orally, transdermally, transmucosally, or rectally.

35. Use of methylnaltrexone for the manufacture of a medicament for the treatment of mu opioid agonist induced immune suppression, wherein infection by macrophage-tropic HIV-1 of CCR5 positive cells of the patient is to be inhibited.

36. Use of methylnaltrexone for the manufacture of a medicament for the treatment of mu opioid agonist induced immune suppression in a patient infected with HIV, Hepatitis C or Cytomegalovirus.

## Patentansprüche

1. Pharmazeutische Zusammensetzung umfassend Methlynaltrexon für die Behandlung von durch opioide Mü-Agonisten induzierter Immunsuppression bei Patienten, wobei die pharmazeutische Zusammensetzung subkutan in einer Dosis, die im Bereich von 0,001 bis 5 mg/kg, auf das Körpergewicht des Patienten bezogen, liegt, oder enteral, intravenös, oral, transdermal, transmukosal oder rektal verabreicht werden soll.

2. Zusammensetzung nach Anspruch 1, wobei der Patient mindestens ein weiteres pharmazeutisches Mittel erhält, das kein Opioid oder opioider Antagonist ist.

3. Zusammensetzung nach Anspruch 2, wobei das pharmazeutische Mittel ein antivirales Mittel ist.

4. Zusammensetzung nach Anspruch 3, wobei das antivirale Mittel ein antiretrovirales Mittel ist.

5. Zusammensetzung nach Anspruch 3, wobei das antivirale Mittel ein Proteaseinhibitor ist.

6. Zusammensetzung nach Anspruch 3, wobei das antivirale Mittel ein Nucleosidanalog oder Nucleotidanalog ist.

7. Zusammensetzung nach Anspruch 2, wobei das pharmazeutische Mittel aus der Gruppe ausgewählt ist bestehend aus einem infektionsverhindernden Mittel, einem Antikrebsmittel oder einem blutbildenden Stimulationsmittel.

8. Zusammensetzung nach einem der Ansprüche 1 bis 7, wobei der Patient immunsupprimiert ist.

9. Zusammensetzung nach Anspruch 1, die intravenös in einer Dosis im Bereich von 0,001 bis 5 mg/kg, auf das Körpergewicht des Patienten bezogen, verabreicht werden soll.

10. Zusammensetzung nach Anspruch 1, die intravenös in einer Dosis im Bereich von 0,05 bis 0,5 mg/kg, auf das Körpergewicht des Patienten bezogen, verabreicht werden soll.

11. Zusammensetzung nach Anspruch 1, die subkutan in einer Dosis im Bereich von 0,05 bis 0,5 mg/kg, auf das Körpergewicht des Patienten bezogen, verabreicht werden soll.

12. Zusammensetzung nach Anspruch 1, die oral in einer Dosis im Bereich von 1 bis 80 mg/kg, auf das Körpergewicht des Patienten bezogen, verabreicht werden soll.

13. Zusammensetzung nach Anspruch 12, die oral in einer Dosis im Bereich von 2 bis 20 mg/kg, auf das Körpergewicht des Patienten bezogen, verabreicht werden soll.

14. Zusammensetzung nach einem der Ansprüche 1, 12 oder 13, wobei die pharmazeutische Zusammensetzung in Form einer enterisch beschichteten Tablette oder Kapsel vorliegt.

15. Zusammensetzung nach Anspruch 1, wobei die pharmazeutische Zusammensetzung Methylnaltrexon und das Opioid umfasst.

16. Zusammensetzung nach Anspruch 1, wobei die pharmazeutische Zusammensetzung mindestens ein weiteres pharmazeutisches Mittel umfasst.

17. Zusammensetzung nach einem der Ansprüche 1 bis 16, wobei der Patient mit HIV infiziert ist.

18. Zusammensetzung nach Anspruch 17, wobei der Patient an AIDS leidet.

19. Zusammensetzung nach den Ansprüchen 1, 15 - 18, wobei die pharmazeutisch Zusammensetzung Methylnaltrexon in einer Menge umfasst, die wirksam ist, um eine opioidinduzierte Erhöhung der Virusbelastung des Patienten zu verhindern.

20. Zusammensetzung nach Anspruch 19, wobei die Virusbelastung des Patienten überwacht wird.

21. Zusammensetzung nach den Ansprüchen 1, 15 - 18, wobei die pharmazeutische Zusammensetzung Methylnaltrexon in einer Menge umfasst, die wirksam ist, um eine opioidinduzierte Erhöhung der CCR5-Spiegel des Patienten zu verhindern.

22. Zusammensetzung nach Anspruch 21, wobei die CCR5-Spiegel des Patienten überwacht werden.

23. Zusammensetzung nach den Ansprüchen 1, 15 - 18, wobei die pharmazeutische Zusammensetzung Methylnaltrexon in einer Menge umfasst, die wirksam ist, um eine opioidinduzierte Reduktion der Menge an CD4-positiven T-Zellen des Patienten zu verhindern.

24. Zusammensetzung nach Anspruch 23, wobei die Menge an CD4-positiven T-Zellen des Patienten überwacht wird.

25. Zusammensetzung nach einem der Ansprüche 1 bis 24, wobei der Patient Strahlung gegenüber ausgesetzt worden ist.

26. Zusammensetzung nach einem der Ansprüche 1 bis 25, wobei der Patient ein chronischer Opioidkonsument ist.

27. Zusammensetzung nach Anspruch 26, wobei das Opioid Methadon oder Morphin ist.

28. Zusammensetzung nach Anspruch 27, wobei der Patient ein Opioidsüchtiger ist.

29. Zusammensetzung nach Anspruch 1, wobei der Plasmaspiegel von Methylnaltrexon des Patienten 1000 ng/ml, 500 ng/ml, 250 ng/ml, 150 ng/ml, 100 ng/ml oder 50 ng/ml nicht übersteigt.

30. Zusammensetzung nach Anspruch 1 oder 29, wobei die pharmazeutische Zusammensetzung durch eine Methode langsamer Infusion verabreicht werden soll.

31. Zusammensetzung nach Anspruch 30, wobei die pharmazeutische Zusammensetzung Methylnaltrexon und das Opioid umfasst.

32. Methylnaltrexon für die Behandlung von durch opioiden Mü-Agonisten induzierter Immunsuppression, wobei die Infektion von CCR5-positiven Zellen des Patienten durch Makrophagen-tropischem HIV-1 verhindert werden soll.

33. Methylnaltrexon für die Behandlung von durch opioiden Mü-Agonisten induzierter Immunsuppression, bei einem Patienten, der durch HIV, Hepatitis C oder Zytomegalovirus infiziert ist.

34. Verwendung einer pharmazeutischen Zusammensetzung wie in einem der Ansprüche 1 bis 33 definiert, für die Herstellung eines Medikaments für die Behandlung von durch opioiden Mü-Agonisten induzierter Immunsuppression, bei einem Patienten, wobei die pharmazeutische Zusammensetzung subkutan in einer Dosis, die im Bereich von 0,001 bis 5 mg/kg, auf das Körpergewicht des Patienten bezogen, liegt oder enteral, intravenös, oral, transdermal, transmukosal oder rektal verabreicht werden soll.

35. Verwendung von Methylnaltrexon für die Herstellung eines Medikaments für die Behandlung von durch opioiden Mü-Agonisten induzierter Immunsuppression, wobei die Infektion von CCR5-positiven Zellen des Patienten durch Makrophagen-tropisches HIV-1 verhindert werden soll.

36. Verwendung von Methylnaltrexon für die Herstellung eines Medikaments für die Behandlung von durch opioiden Mü-Agonisten induzierter Immunsuppression bei einem Patienten, der mit HIV, Hepatitis C oder Zytomegalovirus infiziert ist.

## Revendications

1. Composition pharmaceutique comprenant de la méthylnaltrexone pour traiter une immunosuppression induite par un agoniste opioïde mu chez un patient, la composition pharmaceutique devant être administrée par voie sous-cutanée à un dosage allant de 0,001 à 5 mg/kg de poids corporel du patient, ou par voie entérale, intraveineuse, orale, transdermique, transmuqueuse ou rectale.

2. Composition selon la revendication 1, ledit patient recevant au moins un autre agent pharmaceutique qui n'est pas un opioïde ou un antagoniste opioïde.

3. Composition selon la revendication 2, l'agent pharmaceutique étant un agent antiviral.

4. Composition selon la revendication 3, l'agent antiviral étant un agent antirétroviral.

5. Composition selon la revendication 3, l'agent antiviral étant un inhibiteur de protéases.

6. Composition selon la revendication 3, l'agent antiviral comprenant un analogue de nucléoside ou un analogue de nucléotide.

7. Composition selon la revendication 2, l'agent pharmaceutique étant choisi dans le groupe constitué par un agent anti-infectieux, un agent anticancéreux ou un agent stimulant hématopoïétique.

8. Composition selon l'une quelconque des revendications 1 à 7, le patient étant immunodéprimé.

9. Composition selon la revendication 1 devant être administrée par voie intraveineuse à un dosage allant de 0,001 à 5 mg/kg de poids corporel du patient.

10. Composition selon la revendication 1 devant être administrée par voie intraveineuse à un dosage allant de 0,05 à 0,5 mg/kg de poids corporel du patient.

11. Composition selon la revendication 1 devant être administrée par voie sous-cutanée à un dosage allant de 0,05 à 0,5 mg/kg de poids corporel du patient.

12. Composition selon la revendication 1 devant être administrée par voie orale à un dosage allant de 1 à 80 mg/kg de poids corporel du patient.

13. Composition selon la revendication 12 devant être administrée par voie orale à un dosage allant de 2 à 20 mg/kg de poids corporel du patient.

14. Composition selon l'une quelconque des revendications 1, 12 ou 13, la composition pharmaceutique étant sous la forme d'un comprimé ou d'une gélule à enrobage gastrorésistant.

15. Composition selon la revendication 1, la composition pharmaceutique comprenant de la méthylnaltrexone et l'opioïde.

16. Composition selon la revendication 1, la composition pharmaceutique comprenant au moins un autre agent pharmaceutique.

17. Composition selon l'une quelconque des revendications 1 à 16, le patient étant infecté par le VIH.

18. Composition selon la revendication 17, le patient ayant le SIDA.

19. Composition selon les revendications 1, 15-18, la composition pharmaceutique comprenant de la méthylnaltrexone en quantité efficace pour inhiber une augmentation induite par un opioïde de la charge virale du patient.

20. Composition selon la revendication 19, la charge virale du patient étant surveillée.

21. Composition selon les revendications 1, 15-18, la composition pharmaceutique comprenant de la méthylnaltrexone en quantité efficace pour inhiber une augmentation induite par un opioïde des taux de CCR5 du patient.

22. Composition selon la revendication 21, les taux de CCR5 du patient étant surveillés.

23. Composition selon les revendications 1, 15-18, la composition pharmaceutique comprenant de la méthylnaltrexone en quantité efficace pour inhiber une diminution induite par un opioïde de la quantité de lymphocytes T CD4 positifs du patient.

24. Composition selon la revendication 23, la quantité des lymphocytes T CD4 positifs du patient étant surveillée.

25. Composition selon l'une quelconque des revendications 1 à 24, le patient ayant été exposé à un rayonnement.

26. Composition selon l'une quelconque des revendications 1 à 25, le patient étant un utilisateur chronique d'opioïdes.

27. Composition selon la revendication 26, l'opioïde étant la méthadone ou la morphine.

28. Composition selon la revendication 27, le patient étant un toxicomane aux opioïdes.

29. Composition selon la revendication 1, le taux plasmatique de méthylnaltrexone du patient ne dépassant pas 1000 ng/ml, 500 ng/ml, 250 ng/ml, 150 ng/ml, 100 ng/ml ou 50 ng/ml.

30. Composition selon la revendication 1 ou 29, la composition pharmaceutique devant être administrée par une méthode de perfusion lente.

31. Composition selon la revendication 30, la composition pharmaceutique comprenant de la méthylnaltrexone et l'opioïde.

32. Méthylnaltrexone pour traiter une immunosuppression induite par un agoniste opioïde mu, une infection par le VIH-1 macrophage-tropique de cellules CCR5 positives du patient devant être inhibée.

33. Méthylnaltrexone pour traiter une immunosuppression induite par un agoniste opioïde mu chez un patient infecté par le VIH, l'hépatite C ou un cytomégalovirus.

34. Utilisation d'une composition pharmaceutique telle que définie dans l'une quelconque des revendications 1 à 33, pour la fabrication d'un médicament pour le traitement d'une immunosuppression induite par un agoniste opioïde mu chez un patient, la composition pharmaceutique devant être administrée par voie sous-cutanée à un dosage allant de 0,001 à 5 mg/kg de poids corporel du patient, ou par voie entérale, intraveineuse, orale, transdermique, transmuqueuse ou rectale.

35. Utilisation de méthylnaltrexone pour la fabrication d'un médicament pour le traitement d'une immunosuppression induite par un agoniste opioïde mu, une infection par le VIH-1 macrophage-tropique de cellules CCR5 positives du patient devant être inhibée.

36. Utilisation de méthylnaltrexone pour la fabrication d'un médicament pour le traitement d'une immunosuppression induite par un agoniste opioïde mu chez un patient infecté par le VIH, l'hépatite C ou un cytomégalovirus.
